# EUROPEAN PATENT APPLICATION

(11) **EP 2 808 681 A1**
(43) Date of publication of application: **03.12.2014**
(21) Application number: 12866963.7
(22) Date of filing: 20.03.2012
(51) Int. Cl.: G01N 33/497, G01N 33/98

(54) **BREATHALYZER**

(30) Priority: 26.01.2012 KR 20120007484
(71) Applicant: DATECH CO., LTD., Incheon 403-030 (KR)
(72) Inventor: PARK, Kwang Hee, Seoul 134-060 (KR); HWANG, Yoon Sik, Incheon 404-300 (KR)
(74) Representative: Hano, Christian
(86) International application number: PCT/KR2012/001990
(87) International publication number: WO 2013/111925

(57) **Abstract**

The invention is a drunkometer comprising a main body (10) and an alcohol sensor (20) provided in the main body (10) and detecting the concentration of the alcohol gas in the exhaled breath of the tested person, the drunkometer (10) further comprises a gas inlet port (21) provided on one side of the main body (10) and communicating with the alcohol sensor (20), into which a gas outlet port (53) of a portable standard gas cartridge (50) containing a standard alcohol gas with the predetermined concentration is detachably connectable, so that test or correction of the drunkometer can be carried out by connecting the gas outlet port (53) of the portable standard gas cartridge (50) into the gas inlet port (21) of the main body (10), detecting the alcoholic concentration of the standard gas cartridge (50) by the alcohol sensor (20) and comparing the detected value with that of the predetermined concentration of the portable standard gas cartridge (50).

## Description

### Technical Field

The present invention relates to a drunkometer, and more particularly to a drunkometer being capable of correcting a measurement error easily and quickly to achieve an accurate measurement and high reliability.

### Background Art

Usually, a drunkometer comprises an alcohol sensor, a signal processor and a display. The alcohol sensor reacts with the alcohol in the exhaled breath and outputs the electrical energy, which is processed and calculated into the alcoholic concentration in the signal processor, and the calculated alcoholic concentration is displayed in the display. But the drunkometer may incur a measurement error inevitably due to the change of the electrochemical characteristics of the alcohol sensor depending on the service environment and service life, which would result in a reduced reliability of the measurement of the alcoholic concentration, and if the personal drunkometer has this problem, then it will incur disputes between the driver and the police officer owing to discrepancy of the measured results of the personal drunkometer and the police officer's drunkometer.

Therefore, a periodic test is required to determine whether the drunkometer operates accurately, and if the drunkometer is not accurate, then correction of the drunkometer should be implemented. In order to test and correct the drunkometer, the test method to use standard alcohol with the predetermined concentration is generally adopted. But this method to use standard alcohol generally requires expensive technical equipment and safety apparatus, and the test or correction methods are different depending on the manufactures of the drunkometer. As a result, the test or correction of the drunkometer is usually implemented by skilled personnel. Therefore, the test and correction of the drunkometer requires high cost, long time and much effort, so the drunkometer may not be tested and corrected timely, and the inaccurate drunkometer causes troubles between the driver and the police officer.

### Disclosure

### Technical Problem

In order to solve the above-mentioned problems of the conventional art, it is an object of the present invention to provide a drunkometer being capable of correcting a measurement error easily and quickly to achieve an accurate measurement and high reliability of the drunkometer and save the cost and time associated with the correction of the drunkometer without assistance of technical equipment and skilled personnel.

### Technical Solution

To attain the above object of the present invention, according to an aspect of the present invention, there is provided a drunkometer comprising a main body 10 and an alcohol sensor 20 provided in the main body 10 and detecting the concentration of the alcohol gas in the exhaled breath of the tested person, wherein the drunkometer 10 further comprises a gas inlet port 21 provided on one side of the main body 10 and communicating with the alcohol sensor 20, into which a gas outlet port 53 of a portable standard gas cartridge 50 containing a standard alcohol gas with the predetermined concentration is detachably connectable, so that test or correction of the drunkometer should be carried out by connecting the gas outlet port 53 of the portable standard gas cartridge 50 into the gas inlet port 21 of the main body 10, detecting the alcoholic concentration of the standard gas cartridge 50 by the alcohol sensor 20 and comparing the detected value with that of the predetermined concentration of the portable standard gas cartridge 50.

According to another aspect of the present invention, there is provided a drunkometer, wherein the drunkometer further comprises an interface 17 on the main body 10, which detects the gas outlet port 53 of the portable standard gas cartridge 50 being connected to the gas inlet port 21 of the main body 10 and receives the information of the alcoholic concentration of the standard gas contained in the portable standard gas cartridge 50.

According to another aspect of the present invention, there is provided a drunkometer, wherein the interface 17 comprises a plurality of touch switches 18, 19 disposed adjacent to the gas inlet port 21, and the portable standard gas cartridge 50 further includes the press projections 54 to press the touch switches 18, 10 of the drunkometer.

According to yet another aspect of the present invention, there is provided a drunkometer, wherein the gas outlet port 53 is sealed by a soft membrane, and the gas inlet port 21 is formed in the shape of a pin, so that the pin shaped gas inlet port 21 can be inserted into the gas outlet port 53.

### Advantageous Effects

According to the present invention, as the drunkometer includes a gas inlet port 21 which communicates with the alcohol sensor 20 of the main body 10 and is connectable to the gas outlet port 53 of the portable standard gas cartridge 50 containing the standard alcohol gas with the predetermined concentration, the alcohol concentration of the standard gas can be measured by the alcohol sensor 20 of the drunkometer and the test and correction can be carried out by comparing the measured value of the standard gas with that of the predetermined concentration of the standard gas. According to the present invention, the user of the drunkometer can test and correct his or her drunkometer easily and quickly without assistance of technical equipment and skilled personnel and can reduce the cost and time associated with the drunkometer test and correction. And as the drunkometer can be tested and corrected timely, the accuracy and reliability of the drunkometer can be enhanced or kept highly, so drunken driving can be effectively avoided or prevented.

And, as the drunkometer further includes the interface 17 on the main body 10, which can detect the gas outlet port 53 of the portable standard gas cartridge 50 being connected to the gas inlet port 21 of the main body 10 and receives the information of the alcoholic concentration of the standard gas contained in the portable standard gas cartridge 50, it would not be necessary to manually input the information of the alcoholic concentration of the standard gas into the drunkometer, so the operating process of the test and correction is easy and quick.

And the interface 17 is made of a plurality of touch switches 18, 19 which can be pressed by the press projections 54 of the portable standard gas cartridge 50, and the position of the press projections 54 is changed according to the concentration of the standard gas, then the communication of the alcoholic concentration information between the main body 10 and the standard gas cartridge 50 can be easily achieved by simply connecting the standard gas cartridge 50 into the main body 10 without use of expensive equipment.

Moreover, as the gas outlet port 53 of the portable standard gas cartridges 50 is sealed by a soft membrane, and the gas inlet port 21 of the main body 10 is formed in the shape of a pin, the pin shaped gas inlet port 21 of the main body 10 can be easily inserted into the gas outlet port 53 of the standard gas cartridge 50 without leakage of the standard gas to achieve smooth flow of the standard gas.

### Description of the Drawings

The above and other objects and advantages of the invention will become more apparent by describing a preferred embodiment with reference to the accompanying drawings in which:

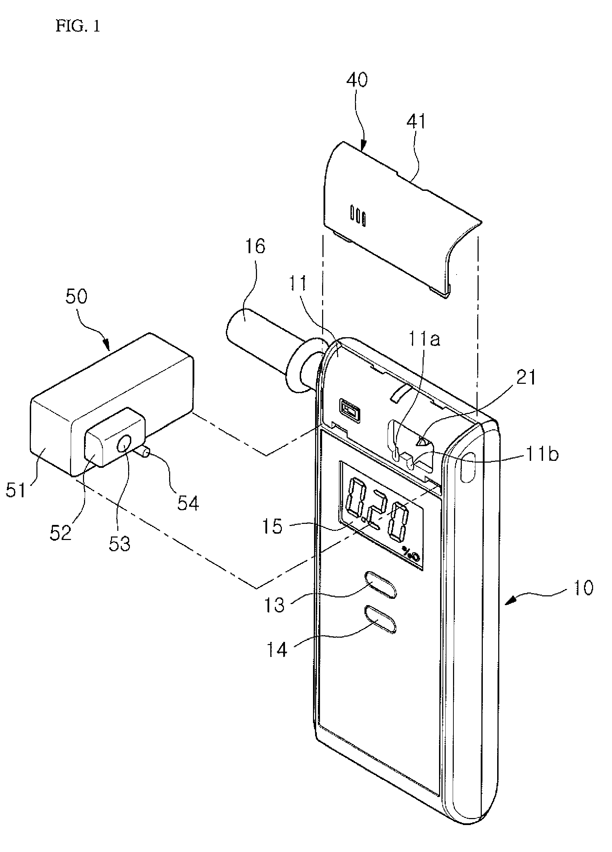

FIG. 1 is an explosive view illustrating an embodiment of the present invention,

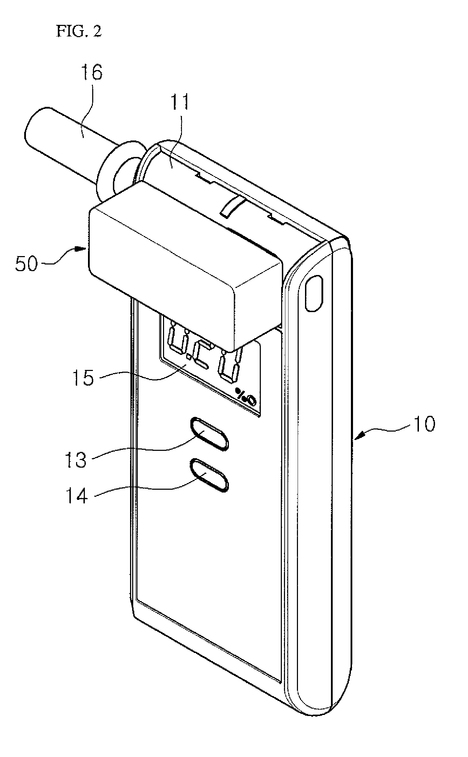

FIG. 2 is perspective view illustrating the above embodiment of the invention,
FIG. 3 is a sectional plan of the above embodiment of the invention,

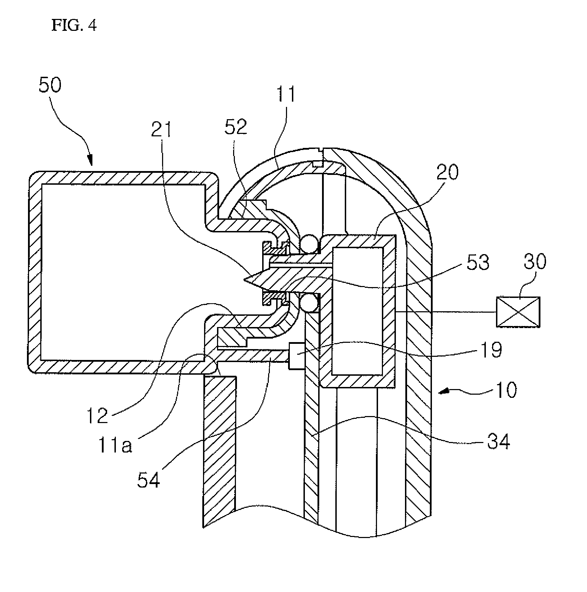

FIG. 4 is a side section of a part of the embodiment of the invention,
FIG. 5 is a flowchart illustrating the operating concept of the invention.

### Detailed Description of the Invention

Hereinbelow, a drunkometer according to a preferred embodiment of the present invention will be described with reference to the accompanying drawings.

The drunkometer of the present invention includes a main body 10 having an exhale tube 16, an alcohol sensor 20 disposed in the main body 10, an interface 17 on one side of the main body 10 and a micom 30 having the program and data to test and correct the drunkometer by using the standard gas cartridge 50 containing the standard alcohol gas with predetermined concentration.

The portable standard gas cartridge 50 includes a cubic shape case 51 of hard material, an inserting projection 52 projected on one side of the case 51, a gas outlet port 53 sealed with membrane of soft material on the front end of the inserting projection 52, and a plurality of press projections 54 adjacent to the inserting projection 52 and projecting in the same direction of the inserting projection 52. The case 51 contains the standard alcohol gas with predetermined concentration, and the information of the concentration of the standard alcohol gas is stored on the storing medium provided on the case 51.

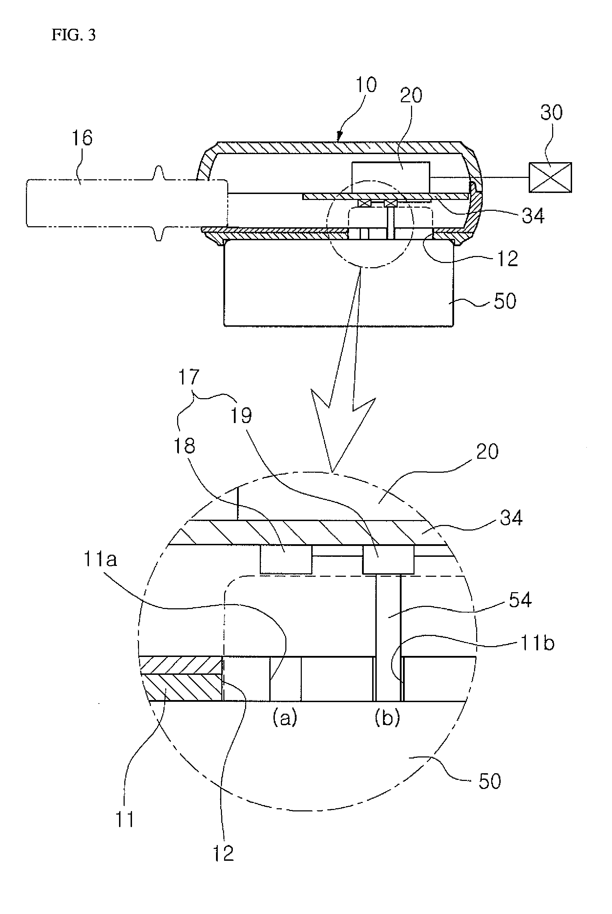

As shown in FIG. 3, the position of the press projections 54 of the standard portable gas cartridge 50 is determined differently depending on the concentration values of the standard alcohol gases. For example, the press projection 54 of the standard gas cartridge 50 containing a standard alcohol gas of 0.05%BAC is positioned on (a), and the press projection 54 of the standard gas cartridge 50 containing a standard alcohol gas of 0.1%BAC is positioned on (b). The press projection 54 of the standard gas cartridge 50 containing a standard alcohol gas of other concentration may be positioned on the other position other than (a) or (b), and the position of the press projection 54 may be variously selected.

The main body 10 is formed in the shape of flat case, and an exhale tube 16 is attached on one side of the upper part of the main body 10. The upper end of the main body 10 is shaped in the rounded form, and a cover mounting recess 11 is formed on the front side of the upper end of the main body 10, on which a cover 40 is detachably mounted. On one side of the cover mounting recess 11 is provided an insert window 12 into which the inserting projection 52 of the portable standard gas cartridge 50 is inserted, and on one side of the insert window 12 is provided a pair of punching holes 11a, 11b corresponding to the positions (a) and (b) of the press projection 54 of the standard gas cartridge 50. The inserting window 12 is preferably formed in the same shape of the inserting projection 52 of the portable standard gas cartridge 50, so that the inserting projection 52 can fit into and seal the inserting window 12 of the main body 10. If necessary, a packing member can be added on the inserting window 12 or the inserting projection 52.

The cover 40 is easily mounted on the cover mounting recess 11 by user's hand. The cover 40 has a cut groove 41, so that the user can easily detach the cover 40 from the main body 10 by use of coin or nail. The main body 10 further includes a power button 13, a mode selection button 14 and a display window 15 showing the concentration of the alcohol on the front surface of it.

The main body 10 includes a solenoid or a motor, and a pump driven by them, and the pump is connected to the alcohol sensor 20 by way of unseen suction pipe. By driving the pump, the alcohol gas introduced from the exhale tube 16 is suctioned to the alcohol sensor 20. Then the alcohol sensor 20 reacts with the introduced alcohol gas to output the electrical energy, and as the output energy is proportional to the alcohol concentration of the alcohol gas, the output energy is processed and calculated into alcohol concentration in the micom 30.

On one side of the alcohol sensor 20 is provided a gas inlet port 21 which is exposed on the inserting window 12 of the main body 10. The gas inlet port 21 is formed in the pin shape, so that it can be easily inserted into the gas outlet port 53 sealed with soft membrane.

The interface 17 receives the information of the alcohol concentration contained in the standard gas cartridge 50 as well as detects the gas outlet port 53 of the standard gas cartridge 50 being connected to the gas inlet port 21 of the main body 10. In this embodiment, the interface 17 comprises touch switches 18, 19 pressed by the press projection 54 disposed on either (a) or (b) position of the standard gas cartridge 50. The first and the second touch switches 18, 19 is provided on the PCB 31 on one side of the gas inlet port 21, and positioned in a pair of punching holes 11a, 11b formed on the cover mounting recess 11 of the main body 10.

When the gas outlet port 53 of the portable standard gas cartridge 50 is connected to the gas inlet port 21 of the main body 10, the press projection 54 positioned in (a) or (b) of the standard gas cartridge 50 will press the first touch switch 18 or the second touch switch 19, then the micom 30 will detect that the gas outlet port 53 of the portable standard gas cartridge 50 is connected to the gas inlet port 21 of the main body 10. The method for the interface 17 of the main body 10 to receives the information of the standard alcohol gas contained in the standard gas cartridge 50 may be the switch type mentioned above, electrical contacts type, RFID, NFC or USB.

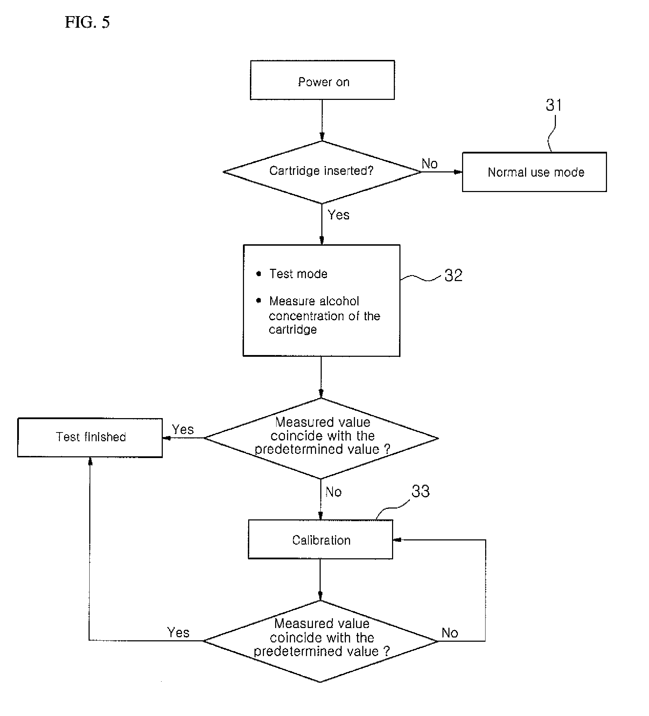

As shown in FIG. 5, when the gas outlet port 53 of the portable standard gas cartridge 50 is connected to the gas outlet port 21 of the main body 10, then the micom 30 receives the electrical signal from the interface 17, and the normal use mode 31 is automatically transferred to the test mode 32. But the user may press the mode selection button 14 to select the test mode 32.

In the normal use mode 31, which is operated when the portable standard gas cartridge 50 is not connected to the main body 10, the alcohol gas in the exhale of the user breath is introduced by the operation of the pump through the exhale tube 16 to the alcohol sensor 20, and reacts with the alcohol sensor 20 to generate the output of electrical energy, which is processed and calculated into alcoholic concentration and displayed in the display window 15.

In the test mode, which is operated when the portable standard gas cartridge 50 is connected to the gas outlet port 21 of the main body 10, the standard alcohol gas in the portable standard gas cartridge 50 is introduced by the operation of the pump to the alcohol sensor 20, and reacts with the alcohol sensor 20 to generate the output of electrical energy, which is processed and calculated into the alcoholic concentration, and it is compared with the predetermined value of concentration of the standard gas cartridge 50. If the measured value of the standard gas coincides with that of the predetermined concentration of the standard gas, that is, the difference is within the allowable error range, the test mode is finished without calibration or correction procedure. If the measured value of the standard gas does not coincide with that of the predetermined concentration of the standard gas, that is, the difference is not within the allowable error range, then the user operates the mode selection button 14 to correction or calibration state 33, in which the calibration action is repeated until the measured value coincides with that of the predetermined concentration of the standard gas. In this embodiment, the calibration state is initiated by operating the mode selection button, but a separate button for calibration may be provided. Meanwhile, after the calibration is finished, it is preferable to proceed to the test mode 32 in order to check whether the correction is normally implemented.

## Claims

1. A drunkometer comprising a main body (10) and an alcohol sensor (20) provided in the main body (10) and detecting the concentration of the alcohol gas in the exhaled breath of the tested person, wherein the drunkometer (10) further comprises a gas inlet port (21) provided on one side of the main body (10) and communicating with the alcohol sensor (20), into which a gas outlet port (53) of a portable standard gas cartridge (50) containing a standard alcohol gas with the predetermined concentration is detachably connectable, so that test or correction of the drunkometer should be carried out by connecting the gas outlet port (53) of the portable standard gas cartridge (50) into the gas inlet port (21) of the main body (10), detecting the alcoholic concentration of the standard gas cartridge (50) by the alcohol sensor (20) and comparing the detected value with that of the predetermined concentration of the portable standard gas cartridge (50).

2. The drunkometer according to claim 1, wherein the drunkometer further comprises an interface (17) on the main body (10), which detects the gas outlet port (53) of the portable standard gas cartridge (50) being connected to the gas inlet port (21) of the main body (10) and receives the information of the alcoholic concentration of the standard gas contained in the portable standard gas cartridge (50).

3. The drunkometer according to claim 2, wherein the interface (17) comprises a plurality of touch switches (18, 19) disposed adjacent to the gas inlet port (21), and the portable standard gas cartridge (50) further includes the press projections (54) to press the touch switches (18, 19) of the drunkometer.

4. The drunkometer according to claim 1 or 2, wherein the gas outlet port (53) is sealed by a soft membrane, and the gas inlet port (21) is formed in the shape of a pin, so that the pin shaped gas inlet port (21) can be inserted into the gas outlet port (53).
